# EUROPEAN PATENT SPECIFICATION

(11) **EP 2 475 997 B1**
(45) Date of publication and mention of the grant of the patent: **02.07.2014**
(21) Application number: 10755224.2
(22) Date of filing: 09.09.2010
(51) Int. Cl.: G01N 33/68

(54) **BIMARKERS FOR SCHIZOPHRENIA OR OTHER PSYCHOTIC DISORDERS**
BIOMARKER FÜR SCHIZOPHRENIE ODER ANDERE PSYCHOTISCHE STÖRUNGEN
BIOMARQUEURS POUR SCHIZOPHRÉNIE OU AUTRES DESORDRES PSICHOTIQUES

(30) Priority: 09.09.2009 GB 0915736
(43) Date of publication of application: 18.07.2012
(60) Divisional application: 14170410.6
(73) Proprietor: Cambridge Enterprise Limited, Trinity Lane Cambridge Cambridgeshire CB2 1TN (GB)
(72) Inventor: BAHN, Sabine, Cambridgeshire CB2 1QT (GB); LEVIN, Yishai, Hadera 38523 (IL)
(74) Representative: Gibson, Mark
(86) International application number: PCT/GB2010/051509
(87) International publication number: WO 2011/030153

(56) References cited:
- WO-A1-2009/077763
- WO-A2-2008/090319
- US-A1- 2006 099 593
- US-A1- 2009 175 827
- YANG Y ET AL: "Altered levels of acute phase proteins in the plasma of patients with schizophrenia" ANALYTICAL CHEMISTRY, AMERICAN CHEMICAL SOCIETY, US, vol. 78, 1 June 2006 (2006-06-01), pages 3571-3576, XP002413130 ISSN: 0003-2700 DOI: DOI:10.1021/AC051916X
- JIANG L ET AL: "Proteomic analysis of the cerebrospinal fluid of patients with schizophrenia" AMINO ACIDS, SPRINGER VERLAG, AU, vol. 25, 17 March 2003 (2003-03-17), pages 49-57, XP002413128 ISSN: 0939-4451
- VAWTER M P ET AL: "Application of cDNA microarrays to examine gene expression differences in schizophrenia" BRAIN RESEARCH BULLETIN, ELSEVIER SCIENCE LTD, OXFORD, GB, vol. 55, no. 5, 15 July 2001 (2001-07-15), pages 641-650, XP002271622 ISSN: 0361-9230 DOI: DOI:10.1016/S0361-9230(01)00522-6
- GRASHOF JAN ET AL: "Functional analysis of components of the presynaptic release machinery in temporal lobe epilepsy" EPILEPSIA, vol. 47, no. Suppl. 4, 2006, page 306, XP002607032 & 60TH ANNUAL MEETING OF THE AMERICAN-EPILEPSY-SOCIETY; SAN DIEGO, CA, USA; DECEMBER 01 -05, 2006 ISSN: 0013-9580
- BABITY JOSEPH M ET AL: "A novel seizure-induced synaptotagmin gene identified by differential display" PROCEEDINGS OF THE NATIONAL ACADEMY OF SCIENCES OF THE UNITED STATES OF AMERICA, vol. 94, no. 6, 1997, pages 2638-2641, XP002607033 ISSN: 0027-8424
- GLAVAN ET AL: "Differential expression of striatal synaptotagmin mRNA isoforms in hemiparkinsonian rats" NEUROSCIENCE, NEW YORK, NY, US, vol. 135, no. 2, 1 January 2005 (2005-01-01), pages 545-554, XP005123514 ISSN: 0306-4522 DOI: DOI:10.1016/J.NEUROSCIENCE.2005.05.050

## Description

### FIELD OF THE INVENTION

The invention relates to a method of diagnosing or monitoring schizophrenia or other psychotic disorder.

### BACKGROUND OF THE INVENTION

Schizophrenia is a psychiatric diagnosis that describes a mental disorder characterized by abnormalities in the perception or expression of reality. It most commonly manifests as auditory hallucinations, paranoid or bizarre delusions, or disorganized speech and thinking with significant social or occupational dysfunction. Onset of symptoms typically occurs in young adulthood, with approximately 0.4-0.6% of the population affected. Diagnosis is based on the patient's self-reported experiences and observed behavior. No laboratory test for schizophrenia currently exists.

Studies suggest that genetics, early environment, neurobiology, psychological and social processes are important contributory factors; some recreational and prescription drugs appear to cause or worsen symptoms. Current psychiatric research is focused on the role of neurobiology, but no single organic cause has been found. Due to the many possible combinations of symptoms, there is debate about whether the diagnosis represents a single disorder or a number of discrete syndromes.

The disorder is thought to mainly affect cognition, but it also usually contributes to chronic problems with behavior and emotion. People with schizophrenia are likely to have additional (comorbid) conditions, including major depression and anxiety disorders; the lifetime occurrence of substance abuse is around 40%. Social problems, such as long-term unemployment, poverty and homelessness, are common. Furthermore, the average life expectancy of people with the disorder is 10 to 12 years less than those without, due to increased physical health problems and a higher suicide rate. An important utility of biomarkers for psychotic disorders is their response to medication. Administration of antipsychotics remains a subjective process, relying solely on the experience of clinicians. Furthermore, the development of antipsychotic drugs has been based on chance findings often with little relation to the background driving the observations.

Schizophrenia is treated primarily with antipsychotic medications which are also referred to as neuroleptic drugs or neuroleptics. Newer antipsychotic agents such as Clozapine, Olanzapine, Quetiapine or Risperidone are thought to be more effective in improving negative symptoms of psychotic disorders than older medication like Chlorpromazine. Furthermore, they induce less extrapyramidal side effects (EPS) which are movement disorders resulting from antipsychotic treatment.

The history of neuroleptics dates back to the late 19th century. The flourishing dye industry catalyzed development of new chemicals that lay the background to modern day atypical antipsychotics. Developments in anti malaria, antihistamine and anaesthetic compounds also produced various neuroleptics. The common phenomenon to all these processes is a fundamental lack of understanding of the biological mechanisms and pathways that these drugs affect, apart from the observation that they prominently block D2 receptors in the striatum.

Yang et al (2006) Anal. Chem. 78, 3571-3576 describes a study investigating altered levels of acute phase proteins in the plasma of patients with schizophrenia. US 2006/099593 describes a method for diagnosising schizophrenia using gene expression in mononuclear cells of peripheral blood as an index. Jiang et al (2003) Amino Acids 25, 49-57 describes the proteomic analysis of the cerebrospinal fluid of patients with schizophrenia. WO 2008/090319 describes a method of diagnosing or monitoring a psychotic disorder, or predisposition thereto, comprises measuring, in a sample taken from a subject, the level of a biomarker selected from clusterin precursor, inter α-trypsin inhibitor, IgM, apolipoprotein A2 and α2 H5 glycoprotein. WO 2009/077763 describes a method of diagnosing or monitoring a psychotic disorder, or predisposition thereto, comprises measuring, in a sample taken from a subject, the levels of one or more peptide biomarkers. Vawter et al (2001) Brain Research Bulletin 55(5), 641-650 describes the application of cDNA microarrays to examine gene expression differences in schizophrenia. Grashof et al (2006) Epilepsia 47(4), 306 describes the functional analysis of components of the presynaptic release machinery in temporal lobe epilepsy. Babity et al (1997) Proc. Natl. Acad. Sci. USA 94, 2638-2641 describes the identification of a novel seizure-induced synaptotagmin gene using differential display. Glavan et al (2005) Neuroscience 135, 545-554 describes the differential expression of striatal synaptotagmin mRNA isoforms in hemiparkinsonian rats. US 2009/175827 describes methods and compositions for identifying genes or genetic pathways modulated by miR-16, using miR-16 to modulate a gene or gene pathway, using this profile in assessing the condition of a patient and/or treating the patient with an appropriate miRNA.

There is therefore a pressing need for objective molecular readouts that can diagnose schizophrenia or other psychotic disorders and furthermore indicate whether a patient is responding to medication, as well as for predicting prognosis.

### SUMMARY OF THE INVENTION

According to a first aspect of the invention there is provided the use of Synaptotagmin-10 as a biomarker for schizophrenia or other psychotic disorder, or predisposition thereto.

According to a further aspect of the invention, there is provided a method of diagnosing or monitoring schizophrenia or other psychotic disorder, or predisposition thereto comprising:
(a) quantifying the amounts of the analyte biomarkers as defined herein in a biological sample obtained from an individual; and
(b) comparing the amounts of the analyte biomarkers in the biological sample with the amounts present in a normal control biological sample from a normal subject, such that a difference in the level of the analyte biomarkers in the biological sample is indicative of schizophrenia or other psychotic disorder, or predisposition thereto.

According to a further aspect of the invention, there is provided a method of monitoring efficacy of a therapy in a subject having, suspected of having, or of being predisposed to schizophrenia or other psychotic disorder, comprising:
(a) quantifying the amounts of the analyte biomarkers as defined herein in a biological sample obtained from an individual;
(b) comparing the amounts of the analyte biomarkers in the biological sample with the amounts present in a sample obtained from the individual on a previous occasion, such as prior to commencement of therapy, such that a difference in the level of the analyte biomarkers in the biological sample is indicative of a beneficial effect of the therapy.

A further aspect of the invention is the use of a kit for monitoring or diagnosing schizophrenia or other psychotic disorder, wherein said kit comprises a biosensor capable of detecting and/or quantifying the analyte biomarkers as defined herein.

### BRIEF DESCRIPTION OF THE FIGURES

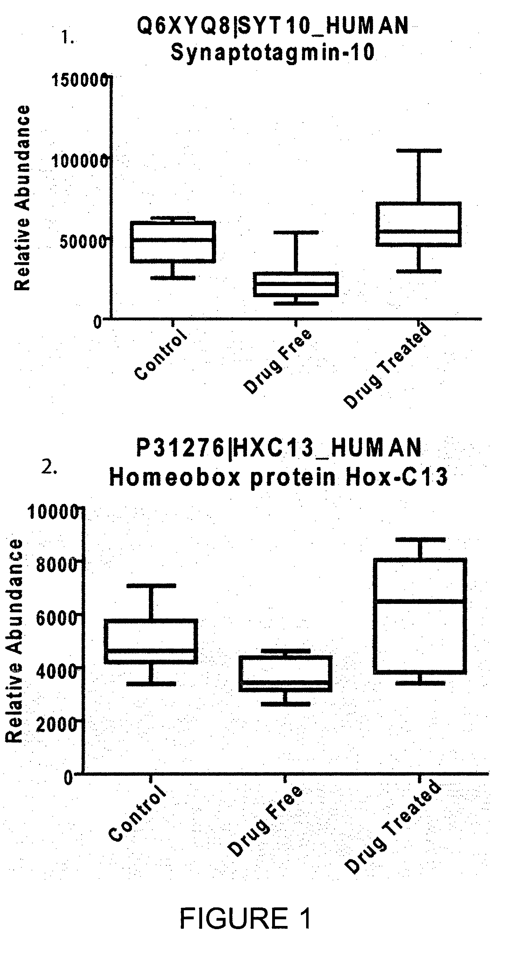

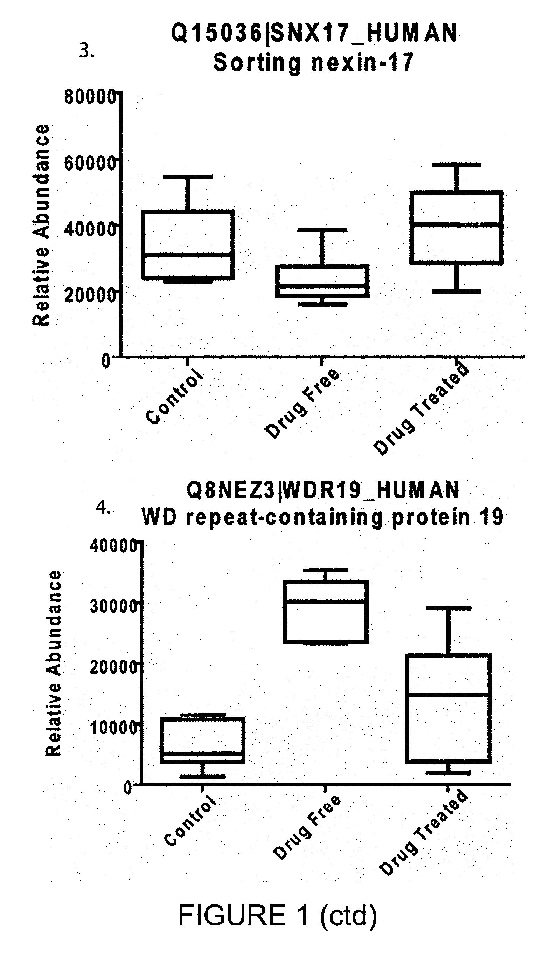

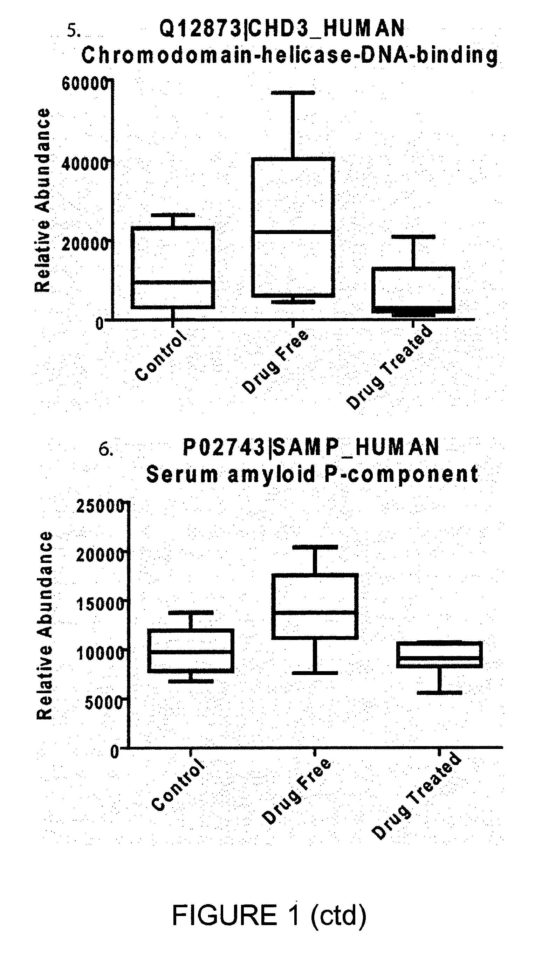

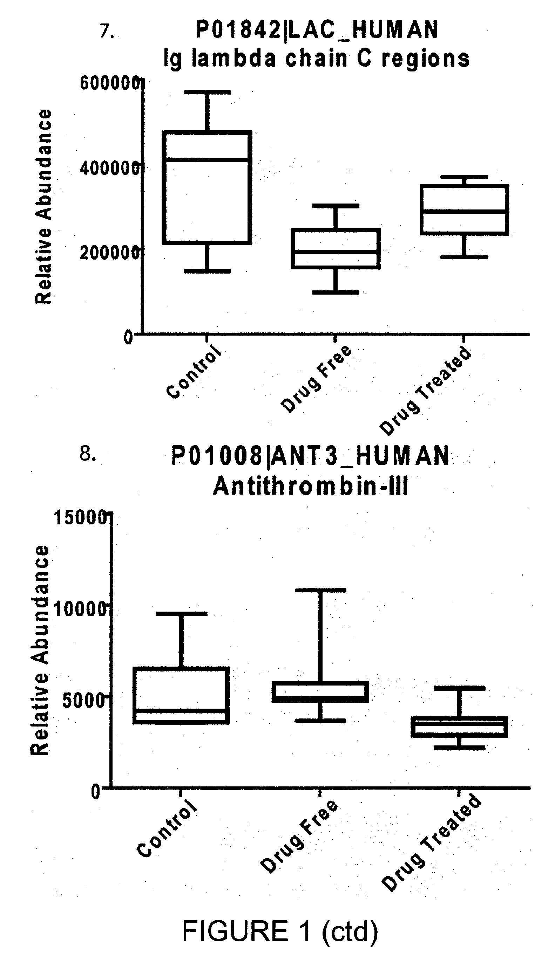

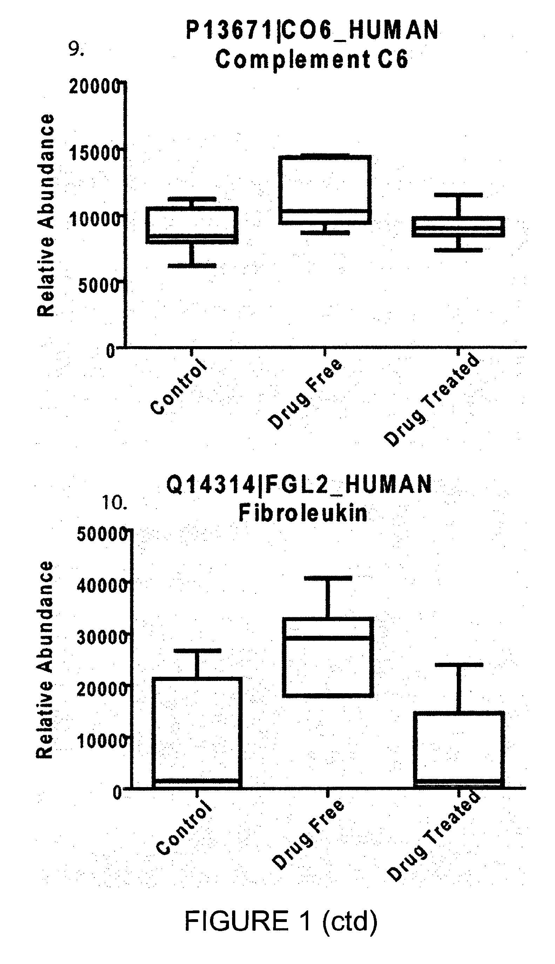

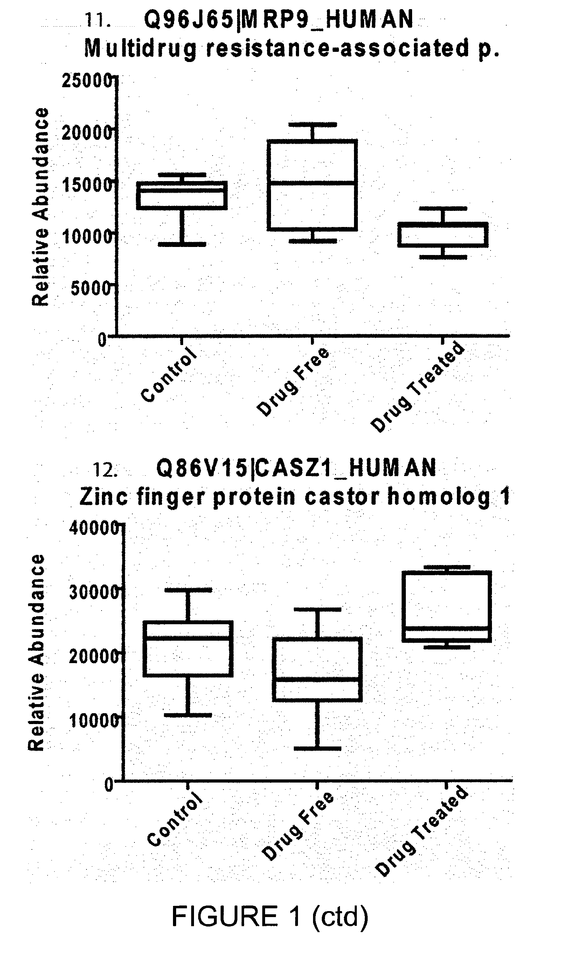

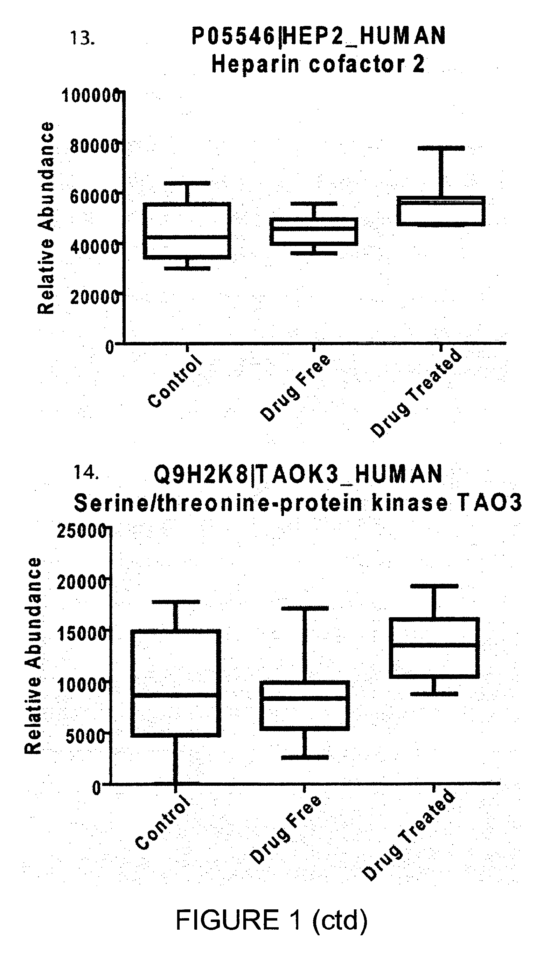

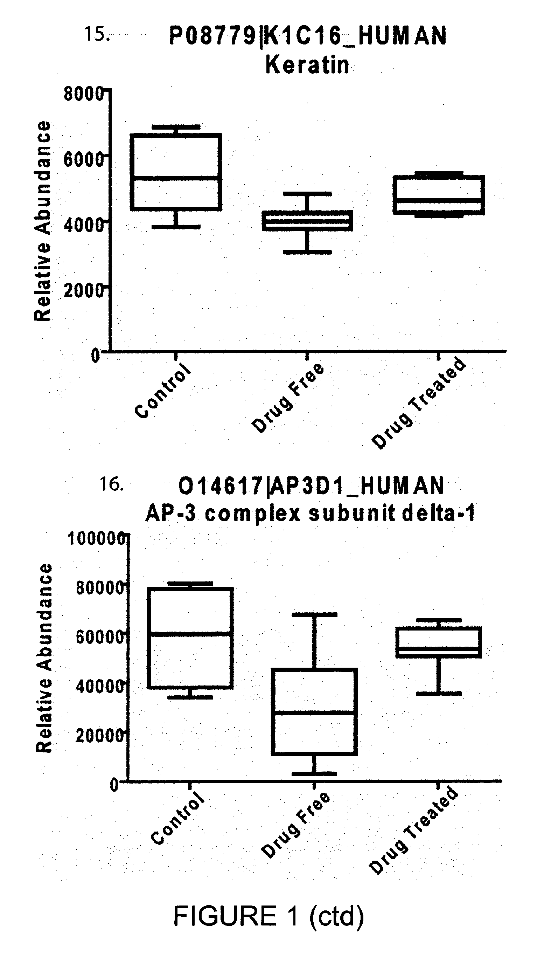

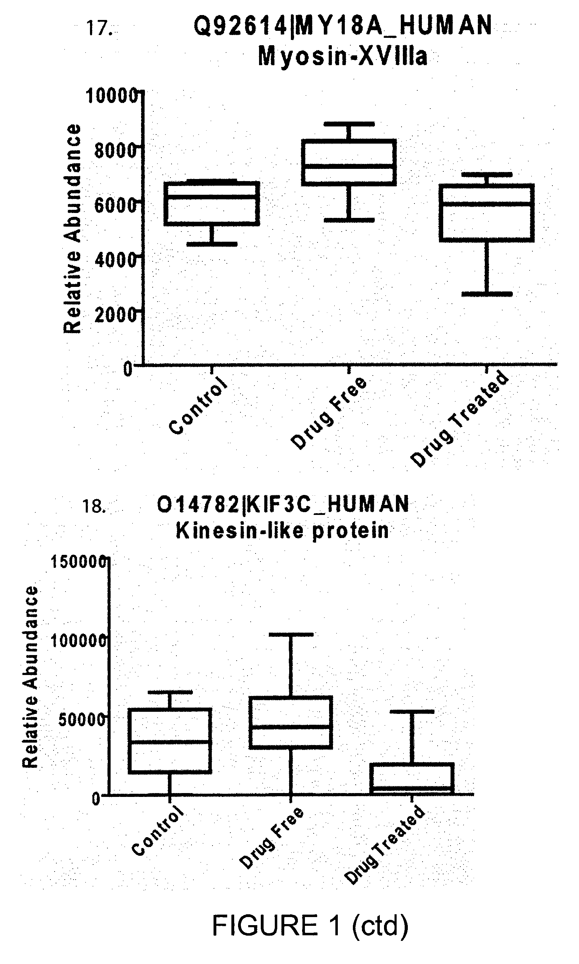

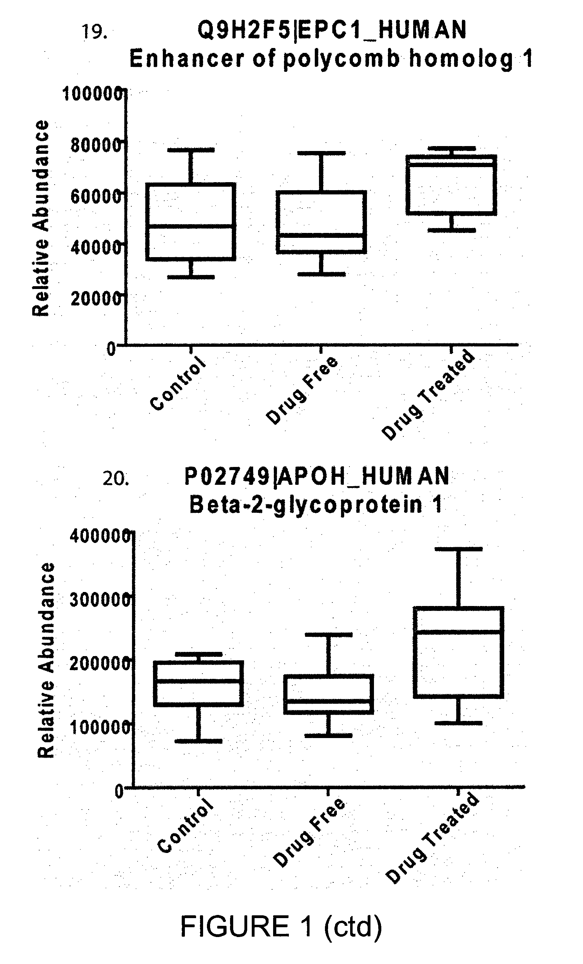

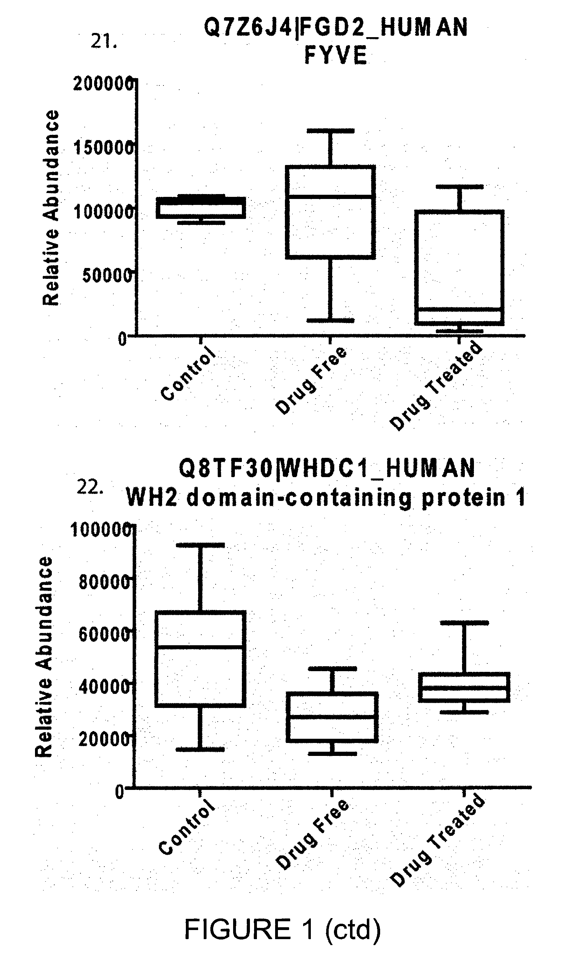

**Figure 1** describes box-plots of the 22 analyte biomarkers of the invention which demonstrated a statistically significant difference in the study described herein.

### DETAILED DESCRIPTION OF THE INVENTION

The term "biomarker" means a distinctive biological or biologically derived indicator of a process, event, or condition. Analyte biomarkers can be used in methods of diagnosis, e.g. clinical screening, and prognosis assessment and in monitoring the results of therapy, identifying patients most likely to respond to a particular therapeutic treatment, drug screening and development. Biomarkers and uses thereof are valuable for identification of new drug treatments and for discovery of new targets for drug treatment.

It will be readily apparent to the skilled person that the analytes listed herein are known and have been described in the literature.

According to a first aspect of the invention there is provided the use of Synaptotagmin-10 as a biomarker for schizophrenia or other psychotic disorder, or predisposition thereto.

The inventors of the present invention have found that Synaptotagmin-10 is down-regulated in samples obtained from schizophrenia patients when compared with samples obtained from a control population. Surprisingly, Synaptotagmin-10 has also been found to be strongly up-regulated following treatment with anti-psychotic medicaments which, without being bound by theory, indicates the presence of a possible "normalization" effect for this biomarker.

In one embodiment, the use additionally comprises the use of one or more additional biomarkers selected from Homeobox protein Hox-C13, WD repeat containing protein 19, Ig lambda chain C regions, Complement C6, Fibroleukin, Multidrug resistance-associated protein 9, Zinc finger protein castor homolog 1, Heparin cofactor 2, AP-3 complex subunit delta-1, Myosin-XVIIIa, Enhancer of polycomb homolog 1, FYVE and WH2 domain-containing protein 1.

According to a further aspect of the invention there is provided the use of Synaptotagmin-10, Homeobox protein Hox-C13, WD repeat containing protein 19, Ig lambda chain C regions, Complement C6, Fibroleukin, Multidrug resistance-associated protein 9, Zinc finger protein castor homolog 1, Heparin cofactor 2, AP-3 complex subunit delta-1, Myosin-XVIIIa, Enhancer of polycomb homolog 1, FYVE and WH2 domain-containing protein 1 as a specific panel of analyte biomarkers for schizophrenia or other psychotic disorder, or predisposition thereto.

In one embodiment, the use additionally comprises one or more additional analytes selected from: Beta-2-glycoprotein 1, Sorting nexin-17, Chromodomain-helicase-DNA-binding protein 3, Serum amyloid P-component, Antithrombin-III, Serine/Threonine-protein kinase TAO3, Keratin and Kinesin-like protein.

According to a further aspect of the invention there is provided the use of Synaptotagmin-10, Homeobox protein Hox-C13, WD repeat containing protein 19, Ig lambda chain C regions, Complement C6, Fibroleukin, Multidrug resistance-associated protein 9, Zinc finger protein castor homolog 1, Heparin cofactor 2, AP-3 complex subunit delta-1, Myosin-XVIIIa, Enhancer of polycomb homolog 1, FYVE, WH2 domain-containing protein 1, Beta-2-glycoprotein 1, Sorting nexin-17, Chromodomain-helicase-DNA-binding protein 3, Serum amyloid P-component, Antithrombin-III, Serine/Threonine-protein kinase TAO3, Keratin and Kinesin-like protein as a specific panel of analyte biomarkers for schizophrenia or other psychotic disorder, or predisposition thereto.

Data is presented in Table 1 and Figure 1 herein which demonstrates that the above mentioned 22 analytes were found to be significantly altered in samples obtained from drug free schizophrenia patients compared with drug treated schizophrenia patients. Therefore, this specific panel of biomarkers is a sensitive and specific predictor for the presence of schizophrenia or other psychotic disorder.

References herein to "other psychotic disorder" relate to any appropriate psychotic disorder according to DSM-IV Diagnostic and Statistical Manual of Mental Disorders, 4th edition, American Psychiatric Assoc, Washington, D.C., 2000. In one particular embodiment, the other psychotic disorder is a psychotic disorder related to schizophrenia. Examples of psychotic disorders related to schizophrenia include brief psychotic disorder delusional disorder, psychotic disorder due to a general medical condition, schizoeffective disorder, schizophreniform disorder, and substance-induced psychotic disorder.

As used herein, the term "biosensor" means anything capable of detecting the presence of the biomarker. Examples of biosensors are described herein.

Biosensors according to the invention may comprise a ligand or ligands, as described herein, capable of specific binding to the analyte biomarker. Such biosensors are useful in detecting and/or quantifying an analyte of the invention.

Diagnostic kits for the diagnosis and monitoring of schizophrenia or other psychotic disorder are described herein which comprise a biosensor capable of detecting and/or quantifying an analyte biomarker.

Monitoring methods of the invention can be used to monitor onset, progression, stabilisation, amelioration and/or remission.

In methods of diagnosing or monitoring according to the invention, detecting and/or quantifying the analyte biomarker in a biological sample from a test subject may be performed on two or more occasions. Comparisons may be made between the level of biomarker in samples taken on two or more occasions. Assessment of any change in the level of the analyte biomarker in samples taken on two or more occasions may be performed. Modulation of the analyte biomarker level is useful as an indicator of the state of schizophrenia or other psychotic disorder or predisposition thereto. An increase in the level of the biomarker, over time is indicative of onset or progression, i.e. worsening of this disorder, whereas a decrease in the level of the analyte biomarker indicates amelioration or remission of the disorder, or vice versa.

A method of diagnosis or monitoring according to the invention may comprise quantifying the analyte biomarker in a test biological sample from a test subject and comparing the level of the analyte present in said test sample with one or more controls.

The control used in a method of the invention can be one or more control(s) selected from the group consisting of: the level of biomarker analyte found in a normal control sample from a normal subject, a normal biomarker analyte level; a normal biomarker analyte range, the level in a sample from a subject with schizophrenia or other psychotic disorder, or a diagnosed predisposition thereto; schizophrenia or other psychotic disorder biomarker analyte level, or schizophrenia or other psychotic disorder biomarker analyte range.

In one embodiment, there is provided a method of diagnosing schizophrenia or other psychotic disorder, or predisposition thereto, which comprises:
(a) quantifying the amount of the analyte biomarker in a test biological sample obtained from an individual; and
(b) comparing the amount of said analyte in said test sample with the amount present in a normal control biological sample from a normal subject.

A higher level of the analyte biomarker in the test sample relative to the level in the normal control is indicative of the presence of schizophrenia or other psychotic disorder, or predisposition thereto; an equivalent or lower level of the analyte in the test sample relative to the normal control is indicative of absence of schizophrenia or other psychotic disorder and/or absence of a predisposition thereto.

The term "diagnosis" as used herein encompasses identification, confirmation, and/or characterisation of schizophrenia or other psychotic disorder, or predisposition thereto. By predisposition it is meant that a subject does not currently present with the disorder, but is liable to be affected by the disorder in time. Methods of monitoring and of diagnosis according to the invention are useful to confirm the existence of a disorder, or predisposition thereto; to monitor development of the disorder by assessing onset and progression, or to assess amelioration or regression of the disorder. Methods of monitoring and of diagnosis are also useful in methods for assessment of clinical screening, prognosis, choice of therapy, evaluation of therapeutic benefit, i.e. for drug screening and drug development.

Efficient diagnosis and monitoring methods provide very powerful "patient solutions" with the potential for improved prognosis, by establishing the correct diagnosis, allowing rapid identification of the most appropriate treatment (thus lessening unnecessary exposure to harmful drug side effects), reducing relapse rates.

Also provided is a method of monitoring efficacy of a therapy for schizophrenia or other psychotic disorder in a subject having such a disorder, suspected of having such a disorder, or of being predisposed thereto, comprising detecting and/or quantifying the analyte present in a biological sample from said subject. In monitoring methods, test samples may be taken on two or more occasions. The method may further comprise comparing the level of the biomarker(s) present in the test sample with one or more control(s) and/or with one or more previous test sample(s) taken earlier from the same test subject, e.g. prior to commencement of therapy, and/or from the same test subject at an earlier stage of therapy. The method may comprise detecting a change in the level of the biomarker(s) in test samples taken on different occasions.

The invention provides a method for monitoring efficacy of therapy for schizophrenia or other psychotic disorder in a subject, comprising:
(a) quantifying the amount of the analyte biomarker; and
(b) comparing the amount of said analyte in said test sample with the amount present in one or more control(s) and/or one or more previous test sample(s) taken at an earlier time from the same test subject.

A decrease in the level of the analyte biomarker in the test sample relative to the level in a previous test sample taken earlier from the same test subject is indicative of a beneficial effect, e.g. stabilisation or improvement, of said therapy on the disorder, suspected disorder or predisposition thereto.

Methods for monitoring efficacy of a therapy can be used to monitor the therapeutic effectiveness of existing therapies and new therapies in human subjects and in non-human animals (e.g. in animal models). These monitoring methods can be incorporated into screens for new drug substances and combinations of substances.

Suitably, the time elapsed between taking samples from a subject undergoing diagnosis or monitoring will be 3 days, 5 days, a week, two weeks, a month, 2 months, 3 months, 6 or 12 months. Samples may be taken prior to and/or during and/or following an anti-psychotic therapy. Samples can be taken at intervals over the remaining life, or a part thereof, of a subject.

The term "detecting" as used herein means confirming the presence of the analyte biomarker present in the sample. Quantifying the amount of the biomarker present in a sample may include determining the concentration of the analyte biomarker present in the sample. Detecting and/or quantifying may be performed directly on the sample, or indirectly on an extract therefrom, or on a dilution thereof.

In alternative aspects of the invention, the presence of the analyte biomarker is assessed by detecting and/or quantifying antibody or fragments thereof capable of specific binding to the biomarker that are generated by the subject's body in response to the analyte and thus are present in a biological sample from a subject having schizophrenia or other psychotic disorder or a predisposition thereto.

Detecting and/or quantifying can be performed by any method suitable to identify the presence and/or amount of a specific protein in a biological sample from a patient or a purification or extract of a biological sample or a dilution thereof. In methods of the invention, quantifying may be performed by measuring the concentration of the analyte biomarker in the sample or samples. Biological samples that may be tested in a method of the invention include cerebrospinal fluid (CSF), whole blood, blood serum, plasma, urine, saliva, or other bodily fluid (stool, tear fluid, synovial fluid, sputum), breath, e.g. as condensed breath, or an extract or purification therefrom, or dilution thereof. Biological samples also include tissue homogenates, tissue sections and biopsy specimens from a live subject, or taken post-mortem. The samples can be prepared, for example where appropriate diluted or concentrated, and stored in the usual manner.

Detection and/or quantification of analyte biomarkers may be performed by detection of the analyte biomarker or of a fragment thereof, e.g. a fragment with C-terminal truncation, or with N-terminal truncation. Fragments are suitably greater than 4 amino acids in length, for example 5, 6, 7, 8, 9, 10, 11, 12, 13, 14, 15, 16, 17, 18, 19, or 20 amino acids in length.

The biomarker may be directly detected, e.g. by SELDI or MALDI-TOF. Alternatively, the biomarker may be detected directly or indirectly via interaction with a ligand or ligands such as an antibody or a biomarker-binding fragment thereof, or other peptide, or ligand, e.g, aptamer, or oligonucleotide, capable of specifically binding the biomarker. The ligand may possess a detectable label, such as a luminescent, fluorescent or radioactive label, and/or an affinity tag.

For example, detecting and/or quantifying can be performed by one or more method(s) selected from the group consisting of: SELDI (-TOF), MALDI (-TOF), a 1-D gel-based analysis, a 2-D gel-based analysis, Mass spec (MS), reverse phase (RP) LC, size permeation (gel filtration), ion exchange, affinity, HPLC, UPLC and other LC or LC MS-based techniques. Appropriate LC MS techniques include ICAT® (Applied Biosystems, CA, USA), or iTRAQ® (Applied Biosystems, CA, USA). Liquid chromatography (e.g. high pressure liquid chromatography (HPLC) or low pressure liquid chromatography (LPLC)), thin-layer chromatography, NMR (nuclear magnetic resonance) spectroscopy could also be used.

Methods of diagnosing or monitoring according to the invention may comprise analysing a sample of cerebrospinal fluid (CSF) by SELDI TOF or MALDI TOF to detect the presence or level of the analyte biomarker. These methods are also suitable for clinical screening, prognosis, monitoring the results of therapy, identifying patients most likely to respond to a particular therapeutic treatment, for drug screening and development, and identification of new targets for drug treatment.

Detecting and/or quantifying the analyte biomarkers may be performed using an immunological method, involving an antibody, or a fragment thereof capable of specific binding to the analyte biomarker. Suitable immunological methods include sandwich immunoassays, such as sandwich ELISA, in which the detection of the analyte biomarkers is performed using two antibodies which recognize different epitopes on a analyte biomarker; radioimmunoassays (RIA), direct, indirect or competitive enzyme linked immunosorbent assays (ELISA), enzyme immunoassays (EIA), Fluorescence immunoassays (FIA), western blotting, immunoprecipitation and any particle-based immunoassay (e.g. using gold, silver, or latex particles, magnetic particles, or Q-dots). Immunological methods may be performed, for example, in microtitre plate or strip format.

Immunological methods in accordance with the invention may be based, for example, on any of the following methods.

Immunoprecipitation is the simplest immunoassay method; this measures the quantity of precipitate, which forms after the reagent antibody has incubated with the sample and reacted with the target antigen present therein to form an insoluble aggregate. Immunoprecipitation reactions may be qualitative or quantitative.

In particle immunoassays, several antibodies are linked to the particle, and the particle is able to bind many antigen molecules simultaneously. This greatly accelerates the speed of the visible reaction. This allows rapid and sensitive detection of the biomarker.

In immunonephelometry, the interaction of an antibody and target antigen on the biomarker results in the formation of immune complexes that are too small to precipitate. However, these complexes will scatter incident light and this can be measured using a nephelometer. The antigen, i.e. biomarker, concentration can be determined within minutes of the reaction.

Radioimmunoassay (RIA) methods employ radioactive isotopes such as I¹²⁵ to label either the antigen or antibody. The isotope used emits gamma rays, which are usually measured following removal of unbound (free) radiolabel. The major advantages of RIA, compared with other immunoassays, are higher sensitivity, easy signal detection, and well-established, rapid assays. The major disadvantages are the health and safety risks posed by the use of radiation and the time and expense associated with maintaining a licensed radiation safety and disposal program. For this reason, RIA has been largely replaced in routine clinical laboratory practice by enzyme immunoassays. Enzyme (EIA) immunoassays were developed as an alternative to radioimmunoassays (RIA). These methods use an enzyme to label either the antibody or target antigen. The sensitivity of EIA approaches that for RIA, without the danger posed by radioactive isotopes. One of the most widely used EIA methods for detection is the enzyme-linked immunosorbent assay (ELISA). ELISA methods may use two antibodies one of which is specific for the target antigen and the other of which is coupled to an enzyme, addition of the substrate for the enzyme results in production of a chemiluminescent or fluorescent signal.

Fluorescent immunoassay (FIA) refers to immunoassays which utilize a fluorescent label or an enzyme label which acts on the substrate to form a fluorescent product. Fluorescent measurements are inherently more sensitive than colorimetric (spectrophotometric) measurements. Therefore, FIA methods have greater analytical sensitivity than EIA methods, which employ absorbance (optical density) measurement.

Chemiluminescent immunoassays utilize a chemiluminescent label, which produces light when excited by chemical energy; the emissions are measured using a light detector.

Immunological methods according to the invention can thus be performed using well-known methods. Any direct (e.g., using a sensor chip) or indirect procedure may be used in the detection of analyte biomarkers of the invention.

The Biotin-Avidin or Biotin-Streptavidin systems are generic labelling systems that can be adapted for use in immunological methods of the invention. One binding partner (hapten, antigen, ligand, aptamer, antibody, enzyme etc) is labelled with biotin and the other partner (surface, e.g. well, bead, sensor etc) is labelled with avidin or streptavidin. This is conventional technology for immunoassays, gene probe assays and (bio)sensors, but is an indirect immobilisation route rather than a direct one. For example a biotinylated ligand (e.g. antibody or aptamer) specific for an analyte biomarker of the invention may be immobilised on an avidin or streptavidin surface, the immobilised ligand may then be exposed to a sample containing or suspected of containing the analyte biomarker in order to detect and/or quantify an analyte biomarker of the invention. Detection and/or quantification of the immobilised antigen may then be performed by an immunological method as described herein.

The term "antibody" as used herein includes, but is not limited to: polyclonal, monoclonal, bispecific, humanised or chimeric antibodies, single chain antibodies, Fab fragments and F(ab')₂ fragments, fragments produced by a Fab expression library, anti-idiotypic (anti-Id) antibodies and epitope-binding fragments of any of the above. The term "antibody" as used herein also refers to immunoglobulin molecules and immunologically-active portions of immunoglobulin molecules, i.e., molecules that contain an antigen binding site that specifically binds an antigen. The immunoglobulin molecules of the invention can be of any class (e. g., IgG, IgE, IgM, IgD and IgA) or subclass of immunoglobulin molecule.

The identification of key biomarkers specific to a disease is central to integration of diagnostic procedures and therapeutic regimes. Using predictive biomarkers appropriate diagnostic tools such as biosensors can be developed; accordingly, in methods and uses of the invention, detecting and quantifying can be performed using a biosensor, microanalytical system, microengineered system, microseparation system, immunochromatography system or other suitable analytical devices. The biosensor may incorporate an immunological method for detection of the biomarker(s), electrical, thermal, magnetic, optical (e.g. hologram) or acoustic technologies. Using such biosensors, it is possible to detect the target biomarker(s) at the anticipated concentrations found in biological samples.

The biomarker(s) of the invention can be detected using a biosensor incorporating technologies based on "smart" holograms, or high frequency acoustic systems, such systems are particularly amenable to "bar code" or array configurations.

In smart hologram sensors (Smart Holograms Ltd, Cambridge, UK), a holographic image is stored in a thin polymer film that is sensitised to react specifically with the biomarker. On exposure, the biomarker reacts with the polymer leading to an alteration in the image displayed by the hologram. The test result read-out can be a change in the optical brightness, image, colour and/or position of the image. For qualitative and semi-quantitative applications, a sensor hologram can be read by eye, thus removing the need for detection equipment. A simple colour sensor can be used to read the signal when quantitative measurements are required. Opacity or colour of the sample does not interfere with operation of the sensor. The format of the sensor allows multiplexing for simultaneous detection of several substances. Reversible and irreversible sensors can be designed to meet different requirements, and continuous monitoring of a particular biomarker of interest is feasible.

Suitably, biosensors for detection of one or more biomarkers of the invention combine biomolecular recognition with appropriate means to convert detection of the presence, or quantitation, of the biomarker in the sample into a signal. Biosensors can be adapted for "alternate site" diagnostic testing, e.g. in the ward, outpatients' department, surgery, home, field and workplace.

Biosensors to detect one or more biomarkers of the invention include acoustic, plasmon resonance, holographic and microengineered sensors. Imprinted recognition elements, thin film transistor technology, magnetic acoustic resonator devices and other novel acousto-electrical systems may be employed in biosensors for detection of the one or more biomarkers of the invention.

Methods involving detection and/or quantification of one or more analyte biomarkers of the invention can be performed on bench-top instruments, or can be incorporated onto disposable, diagnostic or monitoring platforms that can be used in a non-laboratory environment, e.g. in the physician's office or at the patient's bedside. Suitable biosensors for performing methods of the invention include "credit" cards with optical or acoustic readers. Biosensors can be configured to allow the data collected to be electronically transmitted to the physician for interpretation and thus can form the basis for e-neuromedicine.

Any suitable animal may be used as a subject non-human animal, for example a non-human primate, horse, cow, pig, goat, sheep, dog, cat, fish, rodent, e.g. guinea pig, rat or mouse; insect (e.g. *Drosophila*), amphibian (e.g. *Xenopus*) or *C*. *elegans.*

Methods of the invention can be performed in array format, e.g. on a chip, or as a multiwell array. Methods can be adapted into platforms for single tests, or multiple identical or multiple non-identical tests, and can be performed in high throughput format. Methods of the invention may comprise performing one or more additional, different tests to confirm or exclude diagnosis, and/or to further characterise a condition.

The use of a kit for diagnosing or monitoring schizophrenia or other psychotic disorder, or predisposition thereto is provided. Suitably a kit according to the invention may contain one or more components selected from the group: a ligand specific for the analyte biomarker or a structural/shape mimic of the analyte biomarker, one or more controls, one or more reagents and one or more consumables; optionally together with instructions for use of the kit in accordance with any of the methods defined herein.

The identification of biomarkers for schizophrenia or other psychotic disorder permits integration of diagnostic procedures and therapeutic regimes. Currently there are significant delays in determining effective treatment and hitherto it has not been possible to perform rapid assessment of drug response. Traditionally, many anti-psychotic therapies have required treatment trials lasting weeks to months for a given therapeutic approach. Detection of an analyte biomarker of the invention can be used to screen subjects prior to their participation in clinical trials. The biomarkers provide the means to indicate therapeutic response, failure to respond, unfavourable side-effect profile, degree of medication compliance and achievement of adequate serum drug levels. The biomarkers may be used to provide warning of adverse drug response. Biomarkers are useful in development of personalized brain therapies, as assessment of response can be used to fine-tune dosage, minimise the number of prescribed medications, reduce the delay in attaining effective therapy and avoid adverse drug reactions. Thus by monitoring a biomarker of the invention, patient care can be tailored precisely to match the needs determined by the disorder and the pharmacogenomic profile of the patient, the biomarker can thus be used to titrate the optimal dose, predict a positive therapeutic response and identify those patients at high risk of severe side effects.

Biomarker-based tests provide a first line assessment of 'new' patients, and provide objective measures for accurate and rapid diagnosis, in a time frame and with precision, not achievable using the current subjective measures.

Furthermore, diagnostic biomarker tests are useful to identify family members or patients at high risk of developing schizophrenia or other psychotic disorder. This permits initiation of appropriate therapy, or preventive measures, e.g. managing risk factors. These approaches are recognised to improve outcome and may prevent overt onset of the disorder.

Biomarker monitoring methods, biosensors and kits are also vital as patient monitoring tools, to enable the physician to determine whether relapse is due to worsening of the disorder, poor patient compliance or substance abuse. If pharmacological treatment is assessed to be inadequate, then therapy can be reinstated or increased; a change in therapy can be given if appropriate. As the biomarkers are sensitive to the state of the disorder, they provide an indication of the impact of drug therapy or of substance abuse.

The following study illustrates the invention.

The main goal of the present study was the discovery of novel protein biomarkers in blood which respond to drug treatment. These markers could potentially be utilized in a diagnostic test, providing clinicians with objective information during treatment as to an individual patient's response. Furthermore, they could be used to assess compliance of patients with the prescribed drug regime.

The study was conducted by comparing blood samples collected from schizophrenia patients after being off neuroleptics for at least six weeks with follow-up samples collected from the same patients after drug treatment. Treatment response was recorded (based on clinical rating scales). Patients were treated with various combinations of antipsychotics, chosen by a clinician. The approach of combining medication is standard practice among psychiatrists. Comparison of these three groups of samples provides an indication to the feasibility of detecting proteins in the blood that are changing quantitatively as a result of drug treatment.

A total of 30 plasma samples and six QC samples were prepared. These included samples from 10 schizophrenia patients (DSM-IV 295.3) who have been drug free for at least six weeks, 10 plasma samples taken from the same patients after eight weeks of treatment with various anti-psychotics. These patients were selected on the basis of their positive response to treatment, based on clinical ratings. A further 10 samples taken from demographically matched healthy volunteers, were included in the analysis. All samples were collected in the same facility and stored at -80°C. The samples were prepared blind and randomly, without pooling.

Plasma samples were depleted of the 20 most abundant proteins using an immunoaffinity kit. The flow through was injected onto a Strong Anion Exchange column (Propac SAX-10, 4x250mm, Dionex) using the Famos autosampler (LC-Packings/Dionex). A gradient was applied using the Ultimate LC (LC-Packings/Dionex) as follows: 100% A (H₂O + 10mM Na₂HPO₄ for 6min; linear gradient of 85% A in 7 min; linear gradient to 70% A in 25min; linear gradient to 50%A in 2 min; maintain at 50% A for 5 min; return to initial conditions. Buffer B contained: H₂O + 10mM Na₂HPO₄+1.25M NaCl. Five fractions of each sample were collected using the Probot (LC-Packings/Dionex) fraction collector. The fractions were then concentrated using 4ml spin columns (Agilent) with a 5kDa molecular weight cut-off.

Proteins were reduced using 5mM dithriotheitol (Sigma, St. Louis MO) at 60°C for 30min and alkylated with 10mM iodoacetemide (Sigma, St. Louis MO) in the dark at room temperature for 30min. The proteins were digested using Trypsin (Promega, Madison, WI) at a ratio of 1:50 (w/w Trypsin/Protein) for 16 hours at 37°C. The digestion was stopped by adding 2.3µl of 8.8M HCl to each sample. The samples were stored at -80°C until analysis.

In this study three groups were compared: control, drug-free and drug treated. Two pairwise comparisons were performed using an unpaired, two tailed unpaired and paired T-Test (after logarithmic transformation) to identify proteins that show a statistically significant difference (p<0.05) between drug free and drug treated. The 22 proteins which demonstrated a statistically significant difference are shown in Table 1 and Figure 1.

**Table 1**

| Protein | Unpaired T Test (drug free vs treated) | Paired T Test (drug free vs treated) |
|---|---|---|
| Q6XYQ8\|SYT10_HUMAN Synaptotagmin-10 | 0.001 | 0.0066 |
| P31276\|HXC13_HUMAN Homeobox protein Hox-C13 | 0.0065 | 0.0138 |
| Q15036\|SNX17_HUMAN Sorting nexin-17 | 0.0067 | 0.0699 |
| Q8NEZ3\|WDR19_HUMAN WD repeat-containing protein 19 | 0.008 | 0.001 |
| Q12873\|CHD3_HUMAN Chromodomain-helicase-DNA-binding protein 3 | 0.0084 | 0.0418 |
| P02743\|SAMP_HUMAN Serum amyloid P-component | 0.0109 | 0.0621 |
| P01842\|LAC_HUMAN Ig lambda chain C regions | 0.0113 | 0.2821 |
| P01008\|ANT3_HUMAN Antithrombin-III | 0.0138 | 0.1771 |
| P13671\|C06_HUMAN Complement C6 | 0.0141 | 0.0399 |
| Q14314\|FGL2_HUMAN Fibroleukin | 0.017 | 0.1404 |
| Q96J65\|MRP9_HUMAN Multidrug resistance-associated protein 9 | 0.0191 | 0.1013 |
| Q86V15\|CASZ1_HUMAN Zinc finger protein castor homolog 1 | 0.0222 | 0.0206 |
| P05546\|HEP2_HUMAN Heparin cofactor 2 | 0.0242 | 0.0702 |
| Q9H2K8\|TAOK3_HUMAN Serine/threonine-protein kinase TAO3 | 0.0299 | 0.0414 |
| P08779\|K1C16_HUMAN Keratin | 0.0317 | 0.6634 |
| 014617\|AP3D1_HUMAN AP-3 complex subunit delta-1 | 0.0331 | 0.0241 |
| Q92614\|MY18A_HUMAN Myosin-XVIIIa | 0.0344 | 0.1382 |
| 014782\|KIF3C_HUMAN Kinesin-like protein | 0.039 | 0.0763 |
| Q9H2F5\|EPC1_HUMAN Enhancer of polycomb homolog 1 | 0.0436 | 0.1087 |
| P02749\|APOH_HUMAN Beta-2-glycoprotein 1 | 0.0438 | 0.067 |
| Q7Z6J4\|FGD2_HUMAN FYVE | 0.0482 | 0.1127 |
| Q8TF30\|WHDC1_HUMAN WH2 domain-containing protein 1 | 0.1277 | 0.0337 |

## Claims

1. Use of Synaptotagmin-10 as a biomarker for schizophrenia or other psychotic disorder, or predisposition thereto.

2. Use as defined in claim 1, which additionally comprises the use of one or more additional analytes selected from: Homeobox protein Hox-C13, WD repeat containing protein 19, Ig lambda chain C regions, Complement C6, Fibroleukin, Multidrug resistance-associated protein 9, Zinc finger protein castor homolog 1, Heparin cofactor 2, AP-3 complex subunit delta-1, Myosin-XVIIIa, Enhancer of polycomb homolog 1, FYVE and WH2 domain-containing protein 1 as a biomarker for schizophrenia or other psychotic disorder, or predisposition thereto.

3. Use of Synaptotagmin-10, Homeobox protein Hox-C13, WD repeat containing protein 19, Ig lambda chain C regions, Complement C6, Fibroleukin, Multidrug resistance-associated protein 9, Zinc finger protein castor homolog 1, Heparin cofactor 2, AP-3 complex subunit delta-1, Myosin-XVIIIa, Enhancer of polycomb homolog 1, FYVE and WH2 domain-containing protein 1 as a specific panel of analyte biomarkers for schizophrenia or other psychotic disorder, or predisposition thereto.

4. Use as defined in any of claims 1 to 3, which additionally comprises the use of one or more additional analytes selected from: Beta-2-glycoprotein 1, Sorting nexin-17, Chromodomain-helicase-DNA-binding protein 3, Serum amyloid P-component, Antithrombin-III, Serine/Threonine-protein kinase TAO3, Keratin and Kinesin-like protein.

5. Use of Synaptotagmin-10, Homeobox protein Hox-C13, WD repeat containing protein 19, Ig lambda chain C regions, Complement C6, Fibroleukin, Multidrug resistance-associated protein 9, Zinc finger protein castor homolog 1, Heparin cofactor 2, AP-3 complex subunit delta-1, Myosin-XVIIIa, Enhancer of polycomb homolog 1, FYVE, WH2 domain-containing protein 1, Beta-2-glycoprotein 1, Sorting nexin-17, Chromodomain-helicase-DNA-binding protein 3, Serum amyloid P-component, Antithrombin-III, Serine/Threonine-protein kinase TAO3, Keratin and Kinesin-like protein as a specific panel of analyte biomarkers for schizophrenia or other psychotic disorder, or predisposition thereto.

6. A method of diagnosing or monitoring schizophrenia or other psychotic disorder, or predisposition thereto comprising:
(a) quantifying the amounts of the analyte biomarkers as defined in any of claims 1 to 5 in a biological sample obtained from an individual; and
(b) comparing the amounts of the analyte biomarkers in the biological sample with the amounts present in a normal control biological sample from a normal subject, such that a difference in the level of the analyte biomarkers in the biological sample is indicative of schizophrenia or other psychotic disorder, or predisposition thereto.

7. A method of monitoring efficacy of a therapy in a subject having, suspected of having, or of being predisposed to schizophrenia or other psychotic disorder, comprising:
(a) quantifying the amounts of the analyte biomarkers as defined in any of claims 1 to 5 in a biological sample obtained from an individual; and
(b) comparing the amounts of the analyte biomarkers in the biological sample with the amounts present in a sample obtained from the individual on a previous occasion, such as prior to commencement of therapy, such that a difference in the level of the analyte biomarkers in the biological sample is indicative of a beneficial effect of the therapy.

8. A method as defined in claims 6 or claim 7, which is conducted on samples taken on two or more occasions from a test subject.

9. A method as defined in any of claims 6 to 8, further comprising comparing the level of the biomarker present in samples taken on two or more occasions.

10. A method as defined in any of claims 6 to 9, comprising comparing the amount of the biomarker present in said test sample with one or more controls.

11. A method as defined in any of claims 6 to 10, wherein samples are taken prior to and/or during and/or following therapy for schizophrenia or other psychotic disorder.

12. A method as defined in any of claims 6 to 11, wherein quantifying is performed by measuring the concentration of the analyte biomarker in the or each sample.

13. A method as defined in any of claims 6 to 12, wherein detecting and/or quantifying is performed by one or more methods selected from SELDI (-TOF), MALDI (-TOF), a 1-D gel-based analysis, a 2-D gel-based analysis, Mass spec (MS), reverse phase (RP) LC, size permeation (gel filtration), ion exchange, affinity, HPLC, UPLC or other LC or LC-MS-based technique.

14. A method as defined in any of claims 6 to 13, wherein the biological sample is cerebrospinal fluid, whole blood, blood serum, plasma, urine, saliva, or other bodily fluid, or breath, condensed breath, or an extract or purification therefrom, or dilution thereof.

15. Use of a kit for monitoring or diagnosing schizophrenia or other psychotic disorder, wherein said kit comprises a biosensor capable of detecting and/or quantifying the analyte biomarkers as defined in any of claims 1 to 5.

## Patentansprüche

1. Verwendung von Synaptotagmin-10 als Biomarker für Schizophrenie oder eine andere psychotische Störung oder die Veranlagung dafür.

2. Verwendung nach Anspruch 1, die zusätzlich die Verwendung von einem oder mehreren zusätzlichen Analyten beinhaltet, die ausgewählt sind aus:
Homöobox-Protein Hox-C13, WD-Repeat-haltigem Protein 19, Ig-Lambda-Ketten-C-Regionen, Komplement C6, Fibroleukin, Mehrfacharzneimittelresistenz-assoziiertem Protein 9, Zinkfingerprotein-Castor-Homolog 1, Heparin-Cofaktor 2, AP-3-Komplex-Untereinheit Delta-1, Myosin-XVIIIa, Enhancer von Polycomb-Homolog 1, FYVE und
WH2-Domänen-haltigem Protein 1 als Biomarker für Schizophrenie oder eine andere psychotische Störung oder die Veranlagung dafür.

3. Verwendung von Synaptotagmin-10, Homöobox-Protein Hox-C13, WD-Repeat-haltigem Protein 19, Ig-Lambda-Ketten-C-Regionen, Komplement C6, Fibroleukin, Mehrfacharzneimittelresistenz-assoziiertem Protein 9, Zinkfingerprotein-Castor-Homolog 1, Heparin-Cofaktor 2, AP-3-Komplex-Untereinheit Delta-1, Myosin-XVIIIa, Enhancer von Polycomb-Homolog 1, FYVE und WH2-Domänen-haltigem Protein 1 als spezifische Gruppe von Analyt-Biomarkern für Schizophrenie oder eine andere psychotische Störung oder die Veranlagung dafür.

4. Verwendung nach einem der Ansprüche 1 bis 3, die zusätzlich die Verwendung von einem oder mehreren zusätzlichen Analyten beinhaltet, die ausgewählt sind aus: Beta-2-Glykoprotein 1, Sorting-Nexin-17, Chromodomänen-Helikase-DNA-Bindeprotein 3, Serum-Amyloid-P-Komponente, Antithrombin-Ill, Serin/Threonin-Proteinkinase TAO3, Keratin und Kinesin-artigem Protein.

5. Verwendung von Synaptotagmin-10, Homöobox-Protein Hox-C13, WD-Repeat-haltigem Protein 19, Ig-Lambda-Ketten-C-Regionen, Komplement C6, Fibroleukin, Mehrfacharzneimittelresistenz-assoziiertem Protein 9, Zinkfingerprotein-Castor-Homolog 1, Heparin-Cofaktor 2, AP-3-Komplex-Untereinheit Delta-1, Myosin-XVIIIa, Enhancer von Polycomb-Homolog 1, FYVE, WH2-Domänen-haltigem Protein 1, Beta-2-Glykoprotein 1, Sorting-Nexin-17, Chromodomänen-Helikase-DNA-Bindeprotein 3, Serum-Amyloid-P-Komponente, Antithrombin-III, Serin/Threonin-Proteinkinase TAO3, Keratin und Kinesin-artigem Protein als spezifische Gruppe von Analyt-Biomarkern für Schizophrenie oder eine andere psychotische Störung oder die Veranlagung dafür.

6. Verfahren zur Diagnose oder Überwachung von Schizophrenie oder einer anderen psychotischen Störung oder der Veranlagung dafür, das Folgendes beinhaltet:
(a) Quantifizieren der Mengen der Analyt-Biomarker nach einem der Ansprüche 1 bis 5 in einer biologischen Probe, die von einem Individuum erhalten wurde; und
(b) Vergleichen der Mengen der Analyt-Biomarker in der biologischen Probe mit den Mengen, die in einer normalen biologischen Kontrollprobe von einem normalen Subjekt enthalten sind, so dass eine Differenz hinsichtlich des Anteils der Analyt-Biomarker in der biologischen Probe auf Schizophrenie oder eine andere psychotische Störung oder die Veranlagung dafür hindeutet.

7. Verfahren zur Überwachung der Wirksamkeit einer Therapie bei einem Subjekt, das Schizophrenie oder eine andere psychotische Störung hat, vermutlich Schizophrenie oder eine andere psychotische Störung hat oder vermutlich die Veranlagung dafür hat, das Folgendes beinhaltet:
(a) Quantifizieren der Mengen der Analyt-Biomarker nach einem der Ansprüche 1 bis 5 in einer biologischen Probe, die von einem Individuum erhalten wurde; und
(b) Vergleichen der Mengen der Analyt-Biomarker in der biologischen Probe mit den Mengen, die in einer Probe enthalten sind, die von dem Individuum bei einer früheren Gelegenheit, z.B. vor Therapiebeginn, erhalten wurde, so dass eine Differenz hinsichtlich des Anteils der Analyt-Biomarker in der biologischen Probe auf einen vorteilhaften Effekt der Therapie hindeutet.

8. Verfahren nach Anspruch 6 oder Anspruch 7, das an Proben durchgeführt wird, die dem Testsubjekt bei zwei oder mehreren Gelegenheiten entnommen werden.

9. Verfahren nach einem der Ansprüche 6 bis 8, das ferner das Vergleichen des Anteils des Biomarkers beinhaltet, der in bei zwei oder mehreren Gelegenheiten entnommenen Proben enthalten ist.

10. Verfahren nach einem der Ansprüche 6 bis 9, das das Vergleichen der Menge des in der genannten Testprobe enthaltenen Biomarkers mit einer oder mehreren Kontrollen beinhaltet.

11. Verfahren nach einem der Ansprüche 6 bis 10, wobei Proben vor und/oder während und/oder nach der Therapie für Schizophrenie oder eine andere psychotische Störung entnommen werden.

12. Verfahren nach einem der Ansprüche 6 bis 11, wobei die Quantifizierung durch Messen der Konzentration des Analyt-Biomarkers in der oder jeder Probe erfolgt.

13. Verfahren nach einem der Ansprüche 6 bis 12, wobei das Detektieren und/oder Quantifizieren mit einem oder mehreren Verfahren erfolgt, die ausgewählt sind aus: SELDI (-TOF), MALDI (-TOF), einer 1D-gelbasierten Analyse, einer 2D-gelbasierten Analyse, Massenspektrometrie (MS), Umkehrphasen-(RP)-LC, Größenpermeation (Gelfiltration), Ionenaustausch, Affinität, HPLC, UPLC oder einer anderen LC- oder LC-MS-basierten Methode.

14. Verfahren nach einem der Ansprüche 6 bis 13, wobei die biologische Probe Rückenmarksflüssigkeit, Vollblut, Blutserum, Plasma, Urin, Speichel oder eine andere Körperflüssigkeit oder Atem, kondensierter Atem oder ein Extrakt oder Reinigungsprodukt davon oder ein Verdünnungsprodukt davon ist.

15. Verwendung eines Kits zur Überwachung oder Diagnose von Schizophrenie oder einer anderen psychotischen Störung, wobei der genannte Kit einen Biosensor beinhaltet, der die Analyt-Biomarker nach einem der Ansprüche 1 bis 5 detektieren und/oder quantifizieren kann.

## Revendications

1. Utilisation de la synaptotagmine-10 comme biomarqueur de la schizophrénie ou d'un autre trouble psychotique ou d'une prédisposition à ce type d'affection.

2. Utilisation selon la revendication 1, qui comprend également l'utilisation d'un ou de plusieurs analytes additionnels sélectionnés parmi les suivants : protéine à homéoboîte Hox-C13, protéine WDR19 *(WD repeat containing protein 19*), régions C de la chaîne lambda des Ig, complément C6, fibroleukine, MRP9 *(multidrug resistance-associated protein 9*), homologue 1 de la protéine à doigt de zinc de ricin, cofacteur II de l'héparine, sous-unité delta-1 du complexe AP-3, myosine XVIIIa, EPc1 *(enhancer of polycomb homolog 1*), protéine à domaine FYVE et protéine 1 à domaine WH2 comme biomarqueurs de la schizophrénie ou d'un autre trouble psychotique ou d'une prédisposition à ce type d'affection.

3. Utilisation de la synaptotagmine-10, de la protéine à homéoboîte Hox-C13, de la protéine WDR19, de régions C de la chaîne lambda des Ig, du complément C6, de la fibroleukine, de la MRP9, de l'homologue 1 de la protéine à doigt de zinc de ricin, du cofacteur II de l'héparine, de la sous-unité delta-1 du complexe AP-3, de la myosine XVIIIa, de l'EPc1, de la protéine à domaine FYVE et de la protéine 1 à domaine WH2 comme batterie spécifique d'analytes biomarqueurs de la schizophrénie ou d'un autre trouble psychotique ou d'une prédisposition à ce type d'affection.

4. Utilisation selon l'une quelconque des revendications 1 à 3, qui comprend également l'utilisation d'un ou de plusieurs analytes additionnels sélectionnés parmi les suivants: bêta2-glycoprotéine 1, SNX17 (*Sorting nexin-17*), CHD3 *(chromodomain-helicase-DNA-binding protein 3*), composant P amyloïde du sérum, antithrombine III, protéine sérine/thréonine kinase TAO3, kératine et protéine apparentée à la kinésine.

5. Utilisation de la synaptotagmine-10, de la protéine à homéoboîte Hox-C13, de la protéine WDR19, de régions C de la chaîne lambda des Ig, du complément C6, de la fibroleukine, de la MRP9, de l'homologue 1 de la protéine à doigt de zinc de ricin, du cofacteur II de l'héparine, de la sous-unité delta-1 du complexe AP-3, de la myosine XVIIIa, de l'EPc1, de la protéine à domaine FYVE, de la protéine 1 à domaine WH2, de la bêta2-glycoprotéine 1, de SNX17, de CHD3, du composant P amyloïde du sérum, de l'antithrombine III, de la protéine sérine/thréonine kinase TAO3, d'une kératine et d'une protéine apparentée à la kinésine comme batterie spécifique d'analytes biomarqueurs de la schizophrénie ou d'un autre trouble psychotique ou d'une prédisposition à ce type d'affection.

6. Méthode de diagnostic ou de suivi de la schizophrénie ou d'un autre trouble psychotique ou d'une prédisposition à ce type d'affection, qui comprend :
(a) la quantification des quantités des analytes biomarqueurs selon l'une quelconque des revendications 1 à 5 dans un échantillon biologique obtenu chez un individu ; et
(b) la comparaison entre les quantités des analytes biomarqueurs mesurées dans l'échantillon biologique et les quantités présentes dans un échantillon biologique contrôle normal obtenu chez un sujet normal, de sorte qu'une différence dans les taux des analytes biomarqueurs dans l'échantillon biologique est indicatrice de la présence d'une schizophrénie ou d'un autre trouble psychotique ou d'une prédisposition à ce type d'affection.

7. Méthode de suivi de l'efficacité d'une thérapie chez un sujet souffrant ou étant suspecté de souffrir de schizophrénie ou d'un autre trouble psychotique ou de présenter une prédisposition à ce type d'affection, qui comprend :
(a) la quantification des quantités des analytes biomarqueurs selon l'une quelconque des revendications 1 à 5 dans un échantillon biologique obtenu chez un individu ; et
(b) la comparaison entre les quantités des analytes biomarqueurs mesurées dans l'échantillon biologique et les quantités présentes dans un échantillon obtenu chez le même individu à une occasion antérieure, par exemple avant l'introduction de la thérapie, de sorte qu'une différence dans les taux des analytes biomarqueurs dans l'échantillon biologique est indicatrice d'un effet bénéfique de la thérapie.

8. Méthode selon la revendication 6 ou la revendication 7, qui est effectuée sur des échantillons prélevés à deux occasions ou plus chez un sujet testé.

9. Méthode selon l'une quelconque des revendications 6 à 8, qui comprend également la comparaison entre les taux du biomarqueur présent dans des échantillons prélevés à deux occasions ou plus.

10. Méthode selon l'une quelconque des revendications 6 à 9, qui comprend la comparaison entre les taux du biomarqueur présent dans ledit échantillon testé et dans un ou plusieurs contrôles.

11. Méthode selon l'une quelconque des revendications 6 à 10, dans laquelle les échantillons sont prélevés avant et/ou pendant et/ou après une thérapie contre la schizophrénie ou un autre trouble psychotique.

12. Méthode selon l'une quelconque des revendications 6 à 11, dans laquelle la quantification est effectuée en mesurant la concentration en l'analyte biomarqueur dans l'échantillon ou dans chaque échantillon.

13. Méthode selon l'une quelconque des revendications 6 à 12, dans laquelle la détection et/ou quantification est effectuée en utilisant une ou plusieurs techniques sélectionnées parmi les suivantes : spectrométrie de masse SELDI-TOF, spectrométrie de masse MALDI-TOF, analyse sur gel en 1 dimension, analyse sur gel en 2 dimensions, spectrométrie de masse (SM), chromatographie liquide (CL) en phase inverse, chromatographie d'exclusion par perméation de gel (filtration sur gel), chromatographie d'échange d'ions, chromatographie d'affinité, HPLC, UPLC ou autre technique reposant sur la CL ou la CL-SM.

14. Méthode selon l'une quelconque des revendications 6 à 13, dans laquelle l'échantillon biologique est le liquide céphalorachidien, le sang entier, le sérum sanguin, le plasma, l'urine, la salive ou un autre liquide corporel, l'haleine, l'haleine condensée ou un extrait ou produit de purification ou dilution de ceux-ci.

15. Utilisation d'un kit pour le suivi ou le diagnostic de la schizophrénie ou d'un autre trouble psychotique, dans laquelle ledit kit comprend un biocapteur capable de détecter et/ou quantifier les analytes biomarqueurs mentionnés à l'une quelconque des revendications 1 à 5.
